# EUROPEAN PATENT SPECIFICATION

(11) **EP 1 636 092 B1**
(45) Date of publication and mention of the grant of the patent: **10.08.2011**
(21) Application number: 04754413.5
(22) Date of filing: 04.06.2004
(51) Int. Cl.: B65B 3/00

(54) **SYRINGE FILLING STATION**
SPRITZENFÜLLSTATION
POSTE DE REMPLISSAGE DE SERINGUE

(30) Priority: 06.06.2003 US 476578 P
(43) Date of publication of application: 22.03.2006
(73) Proprietor: ACIST Medical Systems, Inc., Eden Prairie, MN 55344 (US)
(72) Inventor: HARTMAN, Steven, Commack, NY 11725 (US); CROSS-HANSEN, Alan, Massapequa Park, NY 11762 (US); GODFREY, Clark, East Northport, NY 11731 (US); WILLIAMS, Robert, C., Fort Salonga, NY 11768 (US)
(74) Representative: Hibbert, Juliet Jane Grace
(86) International application number: PCT/US2004/017802
(87) International publication number: WO 2004/108533

(56) References cited:
- WO-A-00/68137
- US-A- 4 998 570
- US-A- 5 647 409
- US-A- 5 647 409
- US-A- 5 911 252
- US-A1- 2002 107 501

## Description

### FIELD OF THE INVENTION

The invention relates generally to the field of syringe filling, and more particularly to a method and apparatus for peristaltic syringe filling.

### BACKGROUND OF THE INVENTION

Many medical procedures require injecting a fluid into a patient using a syringe. In particular, medical fluid injection systems, such as a CT injector uses one or more syringes to inject a measured amount of a fluid into a patient. Before the syringes can be used to inject a fluid, they must first be filled with a fluid. In some instances, filling a syringe may be inexact, inconvenient, and inflexible.

One method of obtaining a loaded syringe is to purchase a syringe that has been pre-loaded with the desired fluid. However, this method has several disadvantages. First, pre-loaded syringes having the desired fluid may not be readily available. Second, if the syringe is available, it may not have the desired amount of fluid. For these and other reasons, pre-loaded syringes may not be desirable.

An alternative method of filling syringes involve connecting an empty syringe to a supply reservoir via a tubing. The syringe plunger is manually withdrawn from the syringe barrel. As the plunger is withdrawn, fluid within the reservoir is drawn into the syringe. This method provides greater flexibility in fluid type and in the amount to be pre-loaded in the syringe. Here, the operator must "eye-ball' the amount of fluid that has entered the syringe to determine when a desired amount of fluid has been transferred to the syringe. Here, the accuracy of the fluid being transferred is dependant on the skill and care of the operator. Additionally, it may be difficult to completely fill the syringe because of air trapped in the tubing. It may also be difficult in forming a necessary vacuum to draw fluid into the syringe. Further, mutually drawing fluid into the barrel may afford the possibility of contamination inside the syringe because the entire reservoir is not utilized to fill the syringe.

US patent no 5647409 describes an automated, syringe filling apparatus for the on-site filling of syringes with a viscoelastic fluid material from a supply reservoir. A housing has a port for receiving the outlet end region of the viscoelastic supply reservoir and a retainer is provided for releasably retaining a conventional syringe to be filled from the viscoelastic supply reservoir. A flexible plastic tube, preferably formed as part of the viscoelastic supply reservoir, is used to interconnect the supply reservoir with a filling end of a syringe retained in the syringe retainer. A peristaltic pump mounted in the housing has a rotating head with a plurality of rollers which force the viscoelastic material through the tube from the supply reservoir into a retained syringe when the tube is locked between the pump head and a back-up member. Electric pump controls include a timer calibrated for causing the automatic filling of the syringe with predetermined amounts of viscoelastic material from the supply reservoir. The controls include a sensor that automatically shuts off the pump when a syringe being filled is filled to a preestablished maximum level. An air pump is provided for pressurizing the viscoelastic supply reservoir to aid in the viscoelastic pumping operation and insure against air voids being formed in a syringe being filled. A corresponding method is disclosed for the on-site filling of a syringe with a viscoelastic fluid form a supply reservoir.

The invention is a syringe filling device that can fill a syringe with a substantially accurate amount of fluid medium. Further, the filling device may be a completely enclosed system that can be easily operated with a single hand. Also, the filling device may be used with a wide variety of fluids such as a contrast medium or flushing fluid.

The syringe filling device comprises a reservoir attachment, a fluid delivery system, a syringe retaining guide, and a pump to move fluid from a reservoir to a syringe. The fluid delivery system is sterile consumable cartridge that is insertable into the filling station and defines a fluid pathway from a reservoir to a syringe. The fluid delivery system also includes a flexible tubing attached to a spike at one end, and a canula at the other. The spike may be inserted into a reservoir and the canula may be at least partially inserted into a syringe. The tubing can be threaded through the syringe filling station and brought into close proximity with the pump. In one embodiment, rotation of the pump moves fluid through the tubing.

The syringe filling station can also include a user interface. In one embodiment, an operator can input commands into the user interface to specify an amount of fluid that is to be dispensed into the syringe. The user interface can be connected to a processor controlling the rate of pumping to accurately fill a syringe with a desired amount of fluid. The filling station can also include sensors that will detect air in the tubing, depletion of the reservoirs, and the like. The sensors can send signals to the processor. The processor in turn, can direct the pump to carry out certain functions depending upon the data received from the sensors. As a result, the filling station can be used to efficiently and accurately fill syringes with a desired amount of fluid.

In one embodiment, the disposable administration cartridge is available in a pre-assembled state so that it can easily be positioned and disposed within the syringe filling station. The administration cartridge helps to ensure that an aseptic or sterile interface is maintained between the reservoir and spike, and between the canula and syringe. The syringe filling station can also include a clear plastic window through which an operator can view the syringe as it is inserted into the syringe retaining guide. As a result, the syringe filling station provides an aseptic or sterile environment for filling a syringe with a medical fluid.

The invention also includes a method of using the syringe filling station. In one embodiment of the invention, the invention comprises the steps of: a) providing a filling station having a reservoir attachment for removably receiving a reservoir, a fluid pump, and a syringe retaining guide; b) providing a fluid delivery system having a projecting member, a canula, and a flexible tubing; c) installing the fluid delivery system into the filling station to define a fluid pathway from the reservoir attachment to the syringe retaining guide; d) connecting a fluid reservoir to the reservoir attachment; e) inserting a syringe into the retaining guide so that at least a portion of the canula is partially inserted into the syringe; and f) pumping the fluid from the reservoir into the syringe.

Thus, among other things, the invention provides an efficient and reliable apparatus and method for filling a syringe with a fluid. Other objects, features, and advantages of the present invention will be apparent to those of ordinary skill in the art in view of the following detailed description of the invention and the accompanying drawings.

### BRIEF DESCRIPTION OF THE SEVERAL VIEWS OF THE DRAWING(S)

Having thus described the invention in general terms, reference will now be made to the accompanying non-limiting drawings, which are not necessarily drawn to scale:
**Figure 1** is an exemplary graphical illustration of a syringe filling station of the present invention;
**Figure 2** is an exemplary schematic illustration of a syringe filling station of the present invention;
**Figure 3** is an exemplary schematic illustration of the syringe filling station depicted in Figure 1 showing a pump;
**Figure 4** is an exemplary schematic illustration of a syringe filling station adapted for a reservoir in the form of a bag;
**Figure 5** is an exemplary schematic illustration of a syringe filling station of the present invention;
**Figure 6** is an exemplary schematic illustration of a syringe filling station of the present invention depicting a pump motor;
**Figure 7** is an exemplary schematic illustration depicting a canula partially inserted into a syringe;
**Figure 8** is an exemplary, graphic illustration depicting a touchscreen of the present invention;
**Figure 9** is an exemplary, block diagram illustrating an electrical design schematic of an embodiment of the filling station;
**Figure 10** is an exemplary, flow diagram describing a process for filling a syringe that is in accordance with the invention; and
**Figure 11** is an exempting flow diagram correlating syringe filling operations with possible functions that could be displayed on the user interface.

### DETAILED DESCRIPTION OF THE INVENTION

The present invention now will be described more fully hereinafter with reference to the accompanying drawings. Indeed, the invention may be embodied in many different forms and should not be construed as limited to the embodiments set forth herein; rather, these embodiments are provided for illustrative purposes only.

In one embodiment, the invention is a syringe filling station that can fill a syringe with a selected or predetermined amount of fluid. The station may include a reservoir outlet for receiving a reservoir; a syringe retaining guide for releasably receiving and securing a syringe; a fluid delivery system defining a fluid path between the reservoir and the syringe; and a pump for moving the fluid from the reservoir to the syringe. In one preferred embodiment, the syringe filling station includes a user interface to input and control the amount of fluid that is delivered to the syringe.

The reservoir can contain fluids that are useful in the medical field, such as a contrast medium. The term "contrast medium" refers generally to any suitable medium that is typically used in the medical field and that can be injected into a patient or subject to highlight selected areas of the patient's body while he/she is being, for example, radiographically scanned. Typically, a contrast medium is used in conjunction with an imaging device to perform medical diagnostic imaging such as CT scans, MRI, ultrasound, etc. Other media that can be used with the injector include, but are not limited to, saline media, flush media, and the like. The filling station in some embodiments is particularly useful for filling syringes with Newtonian flow fluids having viscosities that are typically up to about 100 cps. Typically, the viscosity is from about 0.5 to 100 cps, and preferably from about 1 to 50 cps.

In one exemplary embodiment, the present invention is a device for delivering a fluid into a syringe. The device comprises a reservoir attachment for removably receiving a reservoir having a fluid; a syringe retaining guide for releasably securing a syringe; a consumable administration cartridge defining a fluid pathway between a reservoir and a syringe; and a pump for pumping a fluid from a reservoir through the administration cartridge and into a syringe. The administration cartridge comprises a projecting member for inserting into a reservoir, a flexible tubing having a distal end attached to the projecting member, and a canula attached to the proximal end of the flexible tubing that is adapted for at least partial insertion into a syringe. Preferably, the administration cartridge is available as a unitary cartridge wherein the projecting member, canula, and spike are preassembled for easy installation into the filling station.

In an alternative exemplary embodiment, the invention is a syringe filling station comprising a reservoir having a fluid medium; a fluid delivery system having a projecting member inserted into the reservoir, a flexible tubing having a distal end attached to the projecting member, and a canula attached to the proximal end of the flexible tubing; a syringe having a generally elongated body and a passageway disposed near the discharge region of the syringe, wherein the canula is extending into the passageway so that fluid can be delivered from the reservoir into the syringe; a pump for pumping the fluid from the reservoir into the syringe; and a control unit that is operatively connected to the pump, whereby the control unit can control the pump so that a specific volume of fluid is dispensed into the syringe.

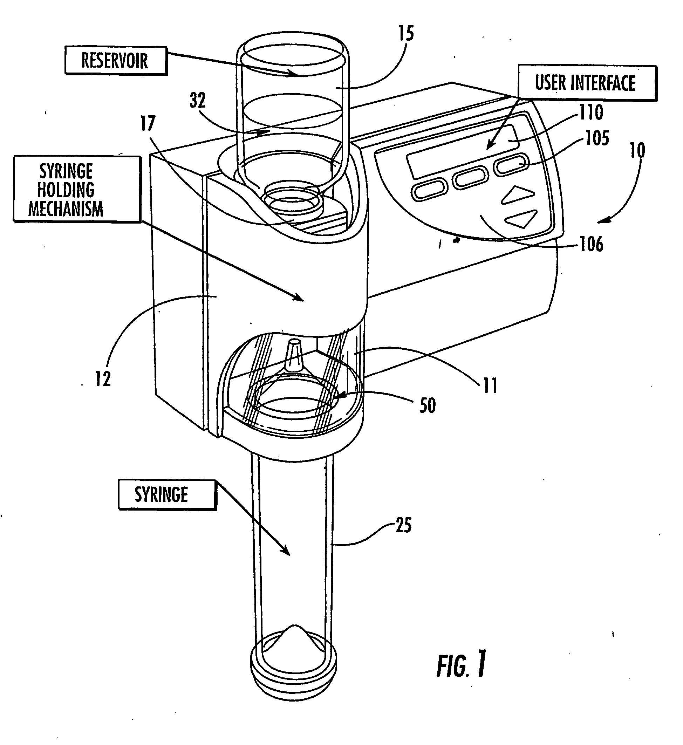

With reference to **FIG.1**, an exemplary syringe filling station is broadly designated with reference number 10. The syringe filling station 10 may include a housing **12** containing a fluid pump (not shown), a reservoir attachment **17,** and a syringe retaining guide **50.** The reservoir attachment can be adapted for removably receiving the reservoir **15.** A syringe retaining lock or guide can be used to guide and securely position the syringe **25** within the housing **12.** In some embodiments, the housing can include a clear viewing window **11** that serves as a visualization aid in assuring that the operator has correctly inserted and attached a syringe. The clear viewing window **11** provides a partial barrier to possible routes of airborne contamination to the tip of a syringe that is disposed in the housing. A fluid delivery system (not shown) defines a fluid path from the reservoir to the syringe. The filling station can also include a user interface **106** having a digital display **110** and touch controls **105** to input syringe filling commands, such as stop, pause, purge line, fill volume, for example.

The syringe filling station as depicted in **FIG.** 1 can be a totally or partially enclosed unit that can be wall-mounted or positioned on a tabletop. The syringe filling station provides a sterile mechanism or conduit for delivering a sterile fluid through a sterile pathway to the interior of a syringe, which may also be sterile.

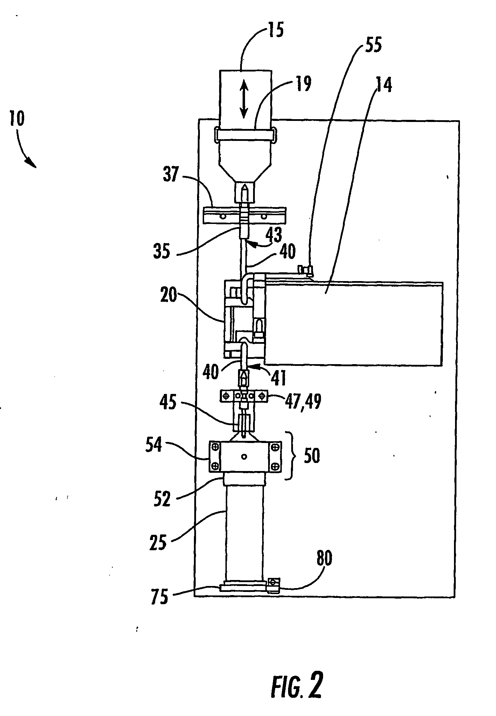

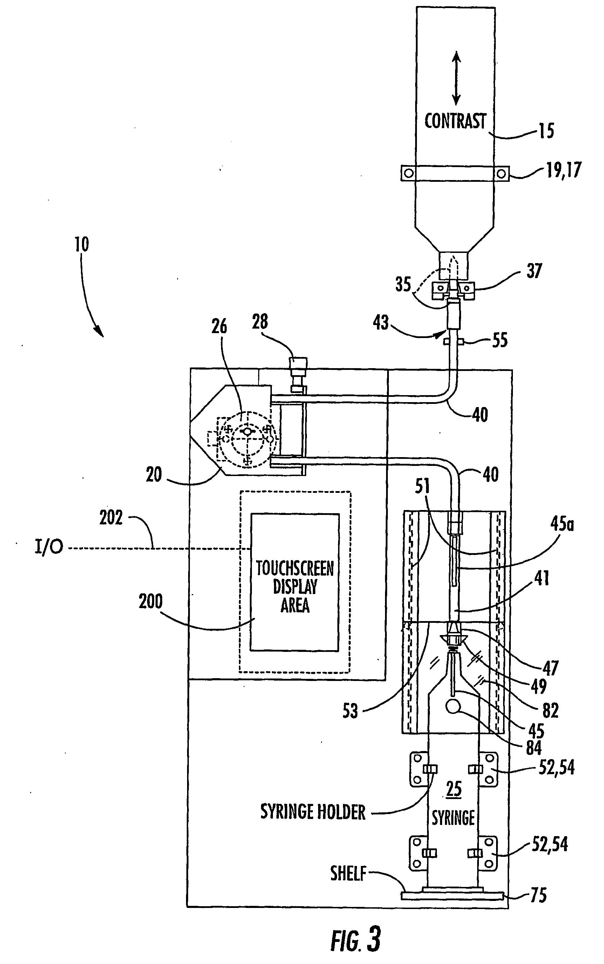

The filling station **10** includes a disposable fluid delivery system that transfers a fluid from the reservoir to the syringe. The fluid delivery system, also called an administration set or cartridge, is a consumable cartridge that can be replaced daily or at some other desirable time. With reference to **FIGS. 2** and **3****,** a schematic illustration of a syringe filling station is shown. The fluid delivery system includes a projecting member **35,** which may include but is not limited to an outwardly projecting member, such as a spike, for example, flexible tubing **40,** and a canula **45.** The spike **35** can be inserted into a reservoir 15. The spike is attached to the upper or distal end **43** of the flexible tubing. The lower or proximal end 41 of the tubing **40** is attached to the canula **45.** The fluid delivery system defines a path through which a fluid can travel from a reservoir to a syringe. In one embodiment, the fluid administration set is installed into the syringe filling station before the reservoir or syringe.

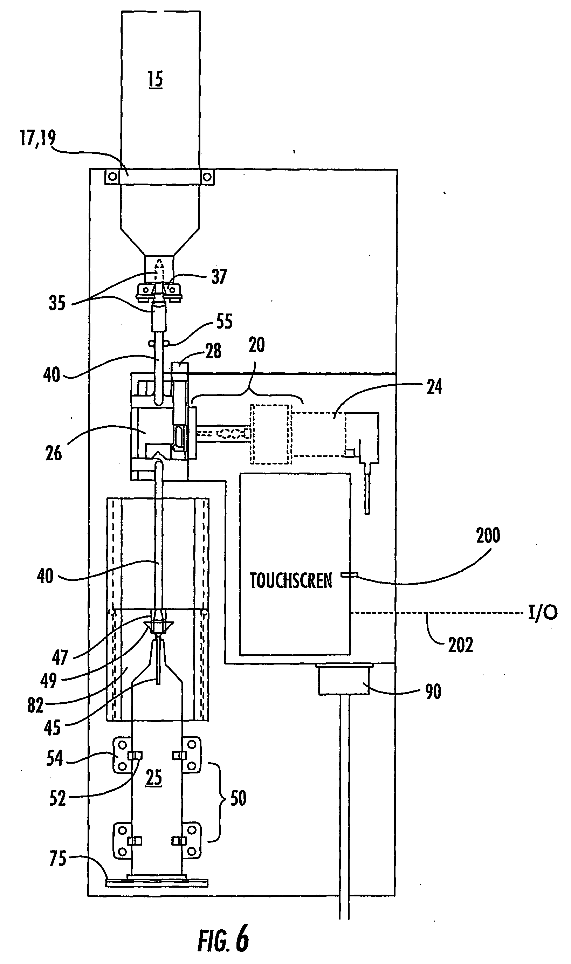

As shown in **FIGS. 2****,** **3****,** and **6****,** the spike can be inserted into the reservoir. The spike is the entry point whereby fluid can enter the administration set and be moved to a syringe. The spike can be vented to allow for the passage of air into the reservoir as fluid is pumped out of the reservoir. The vent may be only one-way so that air cannot exit the reservoir through the vent. The vented spike can be filtered or non-filtered. The spike can be inserted into a standard rubber stoppered bottle or bag, for example. The spike can be supported by a spike locking mechanism or support collar **37.** The spike support collar **37** is typically disposed adjacent to the reservoir **15.** The spike support collar supports the spike and prevents the spike from shifting positions within the reservoir, or accidentally becoming disengaged from the reservoir. After depletion of the reservoir, the emptied reservoir can be easily detached from the spike, and a new reservoir can be attached and connected to the spike with little, if any, physical handling of the spike. As a result, possible contamination may be reduced or limited so that the syringe filling station provides an aseptic environment for syringe filling.

The flexible tubing may extend from the spike **35** to the canula **45.** The tubing may define a fluid pathway through the filling station. Fluid can be moved through the tubing using a pump that applies pressure in a peristaltic fashion to the tubing. The flexible tubing may be composed of any suitable polymeric material so long as the material is flexible, durable, and suitable for medical use.

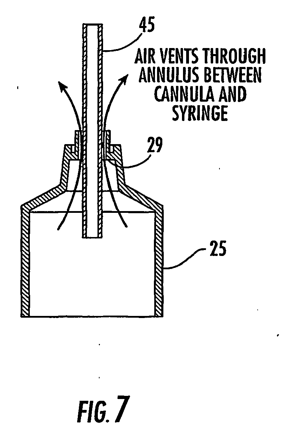

The canula **45** is disposed in close proximity to the syringe retaining guide. The canula typically may have a large bore. **FIGS. 2** and **3** illustrate a guiding mechanism for securely positioning the canula so that it will be positioned in the center of the syringe. As stated above, the canula is typically attached to the lower end of the tubing **40.** The canula can be positioned with a canula mount **47** and a canula guide **49** that are secured to a canula mount housing **53.** The mount housing **53** ensures that the centerline of the syringe opening is coincident with the centerline of the canula. The canula mount **47** and guide **49** can position the canula so that upon insertion of a syringe, the canula will be centered within the syringe (see **FIG. 7**). As a result, the syringe may be easily inserted and aligned within the syringe filling station. In an alternate embodiment, the canula **45,** canula mount **47,** and canula guide **49** can be raised and then lowered into a syringe that has been pre-positioned within syringe retaining guide. In this regard, **FIG. 3** illustrates a canula **45a** in a raised position above a syringe **25.** Once the syringe has been inserted into the retaining guide, the canula **45a** is lowered into the syringe. A linear slide **51** (represented by the dashed lines) can lower and raise the canula **45.** The linear slide is typically lowered or raised using a manual or mechanical mechanism. The syringe filling station can also include a clear viewing window **82** through which an operator can view syringe installation and canula placement. The canula guide and viewing window help to prevent contamination at the point of interface between the canula and the syringe tip. As a result, possible contamination may be limited and an aseptic environment can be maintained while changing and filling syringes.

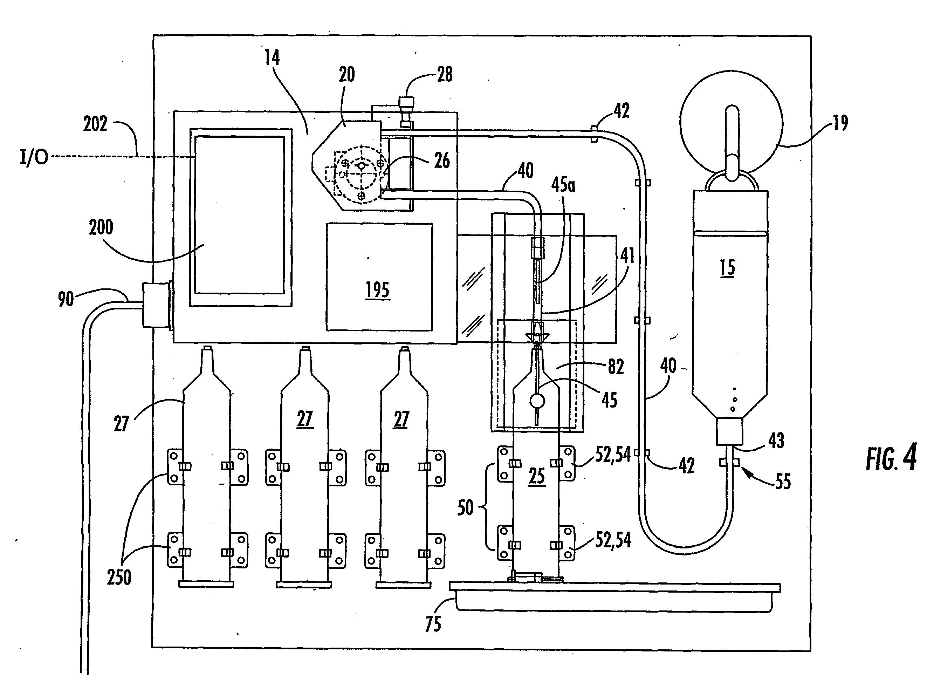

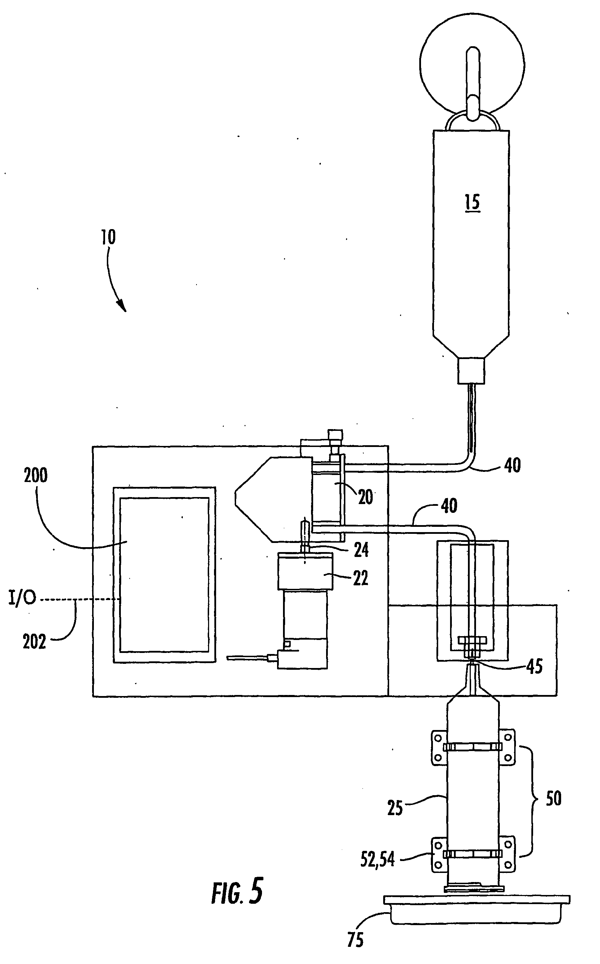

The reservoir can be attached to the filling station in a wide variety of manners. The reservoir attachment should securely position the reservoir, while still allowing the reservoir to be easily removed. As shown in **FIG.1****,** the housing can contain an inlet **17** adapted for removably receiving and connectingly attaching the reservoir **15.** The reservoir can also be secured to the housing with a strap, bracket, sleeve, hook, hanger, or the like, that can removably receive the reservoir. **FIGS. 4** and **5** illustrate a reservoir that is supported and positioned with a reservoir attachment **19** that is comprised of a hanger or hooking mechanism for supporting the reservoir. The reservoir attachment can also include a support pole for securing the bag.

In another embodiment of the present invention, the syringe retaining guide releasably retains the syringe within the filling station. The syringe retaining guide is adapted to positionally secure the syringe within the filling station and to serve as a guide for aligning the syringe with the canula **45.** Preferably, the syringe retaining guide is adapted to securedly lock the syringe in the filling station after it has been installed by an operator. In **FIG. 2**, a syringe retaining guide 50 is illustrated that is comprised of a guiding sleeve 52 and a gripping clip or clamp **54** that can grip and secure the syringe. The guiding sleeve **52** directs the syringe into the filling station so that the canula **45** enters the syringe without touching the inner sidewalls of the syringe. Preferably, the guiding sleeve directs the syringe into the retaining guide so that the canula is also centered within the syringe. The syringe should be positioned within the filling station so that the canula at least partially enters the syringe. The canula should be inserted into the syringe to an extent that prevents spillage of fluid, while still allowing the syringe to be filled and the escape of air from within the syringe. **FIG. 7** illustrates a canula **45** that is partially inserted into the syringe **25.** During the filling process, air can escape from within the syringe through small passageways **29** that exist between the inner wall of the syringe **25** and the canula **45.** The syringe retaining guide **50** can also include brackets, straps, clips, or the like to releasably secure the syringe. In this regard, **FIGS. 3** and **5** illustrate a filling station having two different types of syringe retaining guides that are used to releasably retain the syringe. Typically, the retaining guide will apply pressure to the barrel of the syringe to securely hold the syringe. The retaining guide **50** can also include a syringe support shelf **75** to support the syringe. The shelf can optionally include a pivot member **80** that allows the shelf to pivot about the pivot member. The shelf can also be slidably repositioned. As a result, the shelf can be conveniently moved so that the syringe can be easily inserted into, or withdrawn from, the syringe retaining guide.

The filling station can also include a clear plastic shield 82 that covers the canula **45.** The shield allows an operator to view the insertion of the syringe into the filling station without contaminating the sterile syringe or administration set.

In one embodiment, the fluid pump **20** is used to pump fluid from the reservoir, through the tubing **40** to the canula **45** where it can be dispensed into a syringe. As shown in **FIG. 5****,** the fluid pump **20** may include a peristaltic device **20** having a motor **22,** shaft **24,** and a rotatable peristaltic head **26** (see **FIG.** 3) that applies a pumping pressure to the tubing. In one embodiment, the peristaltic pump can be linear, rotational, or circumferential peristaltic pump. In one embodiment, a DC gear head motor **22** can drive the peristaltic head. In yet another embodiment, the motor is operatively connected to a control unit or processor. The processor may ramp up or ramp down motor speed. Controlling motor speed allows the pumping station to accurately fill a syringe with a desired amount of fluid. The peristaltic device can operate at both constant and variable rates. The fluid pump, control unit, power supply, and processor are normally contained within the housing. As shown in **FIG. 2****,** a removable enclosure **14** covers and encloses the pump and control unit. Power is typically supplied to the pump and control unit with an electric power cord (see **FIG. 4****,** reference number 90). In one embodiment, the pump can move fluid from the reservoir to the syringe without the use of an additional pressure pump.

As shown in **FIG. 3****,** the fluid pump **20** also contains a tightening mechanism **28** that can be loosened to thread the tubing into the pump and around the peristaltic head **26.** As shown in **FIG. 3****,** the tightening mechanism can be a thumb screw that can be easily manipulated. It should be recognized, that the tubing can be secured using a variety of different mechanisms and fasteners. In an alternative embodiment, a lever mechanism for hand operation, or some electro-mechanical mechanism could be used to open and close the geometric gap between the rollers and stator belonging to the peristaltic pump **20** for purposes of loading and unloading the tubing **40.** It is also envisioned that any mechanism or apparatus used to open and close that portion of the peristaltic pump **20** that interfaces with the tubing could include geometric features and attributes to facilitate loading and unloading of tubing. Such geometric features and attributes could similarly interface with some intermediate component attached to or mounted on the exterior of the tube to ease loading and unloading of the tube into the peristaltic pump. Such an arrangement could be as simple as an alignment tap or as complex as a cartridge enclosing the tube that in turn adapts to the peristaltic pump.

The fluid reservoir may contain a wide variety of different fluids such as contrast medias, flushing agents, fluid medications, among others. The syringe filling station is particularly useful for filling syringes with fluids that are used in CT injectors, such as a contrast medium. The fluid reservoir can have the form of a bottle, bag, pouch, container, or the like. The reservoir can also come in a variety of sizes. Typical reservoir volumes can range from about 10 to 1000 cc, with volumes from about 50 to 500 cc being somewhat more preferred. For example, iodinated contrast that is injected to the patient via a CT injector is routinely dispensed in bottles and bags within this range.

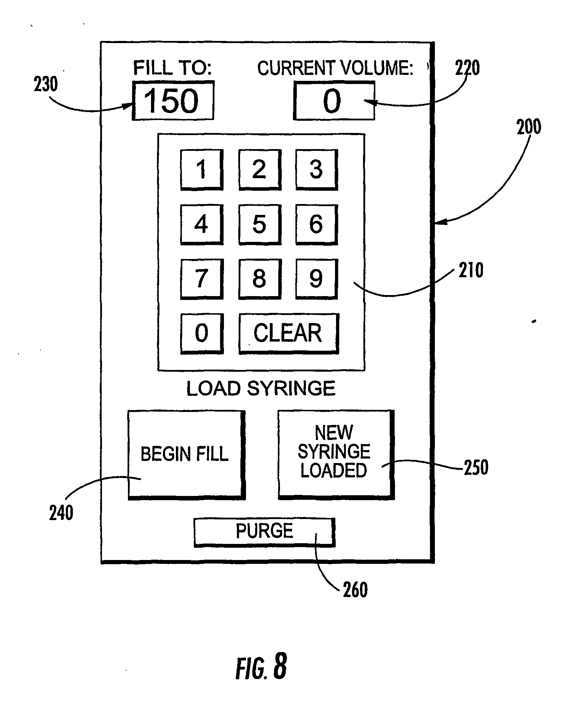

The syringe filling station can include a user interface that an operator can use to control the filling station. Typical command functions that can be entered with the interface include, but are not limited to, Fill volume, New syringe, Begin Fill, Pause, Stop, and Purge. An operator can use the interface to specify the amount of fluid to be dispensed into the syringe. In one embodiment of the present invention, the user interface can be in the form of a touchscreen that allows an operator to input commands. The touchsreen may be incorporated into a PC system that is in communication with the filling station or can be disposed on the filling station housing. In some embodiments, the syringe filling station can be in communication and operatively controlled from a remote interface that could be hosted or displayed, for example, on an injector user interface. The syringe filling station controls could be hosted entirely or partially on an external user interface. In this regard, **FIG. 3** illustrates that the syringe filling station can include an input/output (I/O) terminal **202** for wired or wireless communication. **FIGS. 3** through **6** illustrate a touchsreen **200** that is disposed on the outer surface of the housing **12.** FIG. 8 illustrates an exemplary touchscreen **200** that can be used to operate the filling station. The touchscreen **200** can include several dedicated commands for filling a syringe. The interface can include a dedicated keyboard **210,** or could alternatively include "up" or "down" arrows to vary the volume of fluid dispensed into the syringe. The user interface also includes displays for indicating current syringe volume **220** and the volume to be dispensed **230.** The touchscreen 200 can include dedicated command functions such as "Begin Fill" **240,** "New Syringe Loaded" **250,** and "Purge" **260.** The user interface can contain additional or different dedicated buttons than are described in **FIG. 8****.** In an alternative embodiment, the user interface could be incorporated into an existing injector interface, such as an E-Z-EM Empower CT Remote Control, for example. Alternatively, the syringe filling station data interface could be in communication and operatively connected to an external or remote interface, such as an imaging equipment apparatus or other medical information IT system deployed within an imaging system or hospital. As shown in **FIG. 3****,** the syringe filling station in some embodiments can include an I/O port or data interconnection **202** that can be in communication with an external control system or network. The data interconnection can be wired or wireless. An external interface could be used to input operation commands into the syringe filling station. For example, the external interface could be used to automatically specify syringe fill volumes on a per patient basis. This could help facilitate the tracking of patients electronically through the medical care process. Additionally, such data/control capability could be used to produce hardcopy information from a printing device. Such hardcopy data, particularly in the form of a printed label could be affixed to the syringe for identification purposes as it is removed from the syringe filling station and subsequently handled through injection to the patient. For example, valuable identifying information that could be printed on a label could include, but is not limited to: Date, Time, Person Filling, Patient Name, Patient ID Number, Medical Name of Fluid Filled in Syringe, Brand Name of Fluid Filled in Syringe, Lot Number of Fluid Filled in Syringe, Volume Filled in Syringe, Warnings, Cautions, and the like, and combinations thereof. In some embodiments, an OEM printer could be incorporated into the syringe filling station to provide readily accessible printed labels having the above described information.

The filling station may have a control unit for performing operations. In this regard, **FIG. 4** illustrates a syringe filling station having a control unit **195.** The control unit **195** typically includes a microprocessor that is incorporated into the filling station. The processor can be in communication with the user interface **200** and various sensors. The control unit may include computer code or programs for controlling the filling station and processing information and data received from a user interface, pump, or various sensors. The processor is also operatively connected to pump **20.** In one alternative embodiment of the present invention, the processor can precisely control the pump so that a precise amount of fluid is delivered to the syringe. The pump and processor are normally in communication with each other. The pump can send data, such as pump speed, pumping revolutions, etc, to the processor that can allow the processor to accurately calculate and measure the amount of fluid that has been dispensed into the syringe. In one embodiment, the pump motor can include an encoder. In this embodiment, the volume delivered to the syringe may be measured by counting the pulses of the encoder. During use, the processor will measure the amount of fluid that has been dispensed into the syringe. The processor may also compensate for any air that could be in the administration cartridge. The processor can also optionally contain programmable code that can be used to perform routine system functions and filling operations that are used frequently. For example, if a particular dispensing volume is repeatedly used, the processor can be programmed to deliver a set volume of fluid without requiring the operator to input new fill levels for each new syringe.

When a new administration cartridge is installed into the syringe filling station, the interface may be used to manually or automatically instruct the pump to purge air contained within the tubing.

In another embodiment of the present invention, the filling station may also include sensors that are in communication with the processor and can monitor system status. Such sensors include an air in-line sensor or reservoir empty sensor, a sensor that detects when a new syringe is loaded into the filling station, a sensor for detecting the presence of an administration cartridge, and a sensor for detecting when a new reservoir has been added. The sensors can be used to optimize the performance and efficiency of the syringe filling station.

The air in-line sensor can detect the presence of air in the tubing or the depletion of fluid within the reservoir. The sensor **55** is normally disposed between the reservoir **15** and the pump **20.** As shown in **FIGS. 2** and **3** the sensor **55** can be disposed adjacent to the reservoir. The tubing 40 may pass through the sensor **50.** If a pocket or bubble of air enters into the **tubing** and travels pass the sensor **50,** the sensor can send a signal to the processor indicating the presence of air in the tubing or that the reservoir is depleted. If the sensor detects that the reservoir is depleted, the processor can direct the interface or remote control to display a message that the reservoir is emptied. In one embodiment, operation will not remove until the message has been removed.

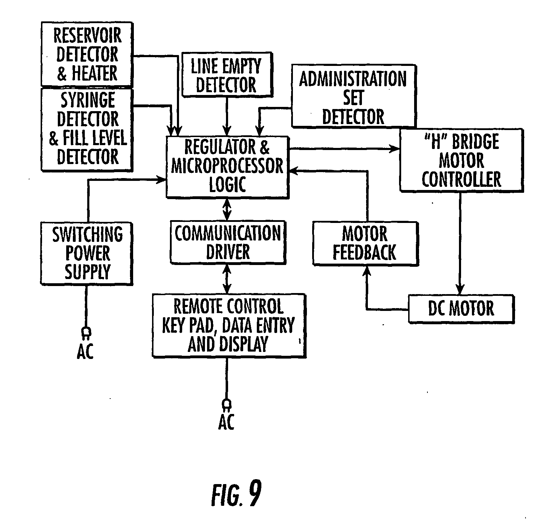

With reference to **FIG. 9****,** an electrical design schematic of one embodiment of the syringe filling station is illustrated. As shown in **FIG. 9****,** the processor performs a variety of functions and is operatively connected to many sensors and devices. The processor can also be in communication with a remote control interface, such as a PC display or other computational CPU that can be used to input commands into the syringe filling station.

The processor can also automatically compensate for the volume of fluid that is needed to purge air from within the tubing. For instance, the processor will compensate for the air in the tubing after a new administration cartridge has been installed. The processor can also compensate for air that will be in the system after a new reservoir has been installed.

The system can also include a timing mechanism that can alert an operator that the administration cartridge needs to be changed. When an operator installs a new administration cartridge into the syringe filling station, a detector positioned within the housing **12,** such as proximal to the syringe retaining guide, can detect the installation of the new cartridge. The sensor can send a signal to the control unit (processor) that a new cartridge has been installed. The processor can then start an internal timer that has been preprogrammed to alert the operator after a specified amount of time that the administration cartridge should be changed. The syringe filing station can also include a sensor to detect when a new syringe has been inserted into the syringe retaining guide. When a new syringe is detected, the processor can direct the interface to reset the syringe volume display to zero. The user interface can also include a command function whereby an operator could input that a new syringe has been installed.

The syringe filling station **10** can also include syringe storage holders **250** for removably storing filled syringes until they are needed. **FIG. 4** illustrates a filling station **10** having a plurality of holding clips for storing filled syringes **27.** The storage holders removably secure the filled syringes. The syringe storage holders can also include an optional heater (not shown) to heat the stored syringes **27** to a temperature that is comfortable for a patient. The filling station **10** can also optionally include a heater for heating the reservoir. The reservoir heater could heat the fluid to a temperature that is comfortable for a patient. The reservoir heater would allow the filled syringe to be used immediately after filling without having to further heat the syringe.

In one embodiment of the invention, the method of filling a syringe with a fluid comprises the steps of connecting a reservoir to a fluid administration cartridge, positioning a canula of the fluid administration cartridge at least partially in a syringe, and moving a fluid from the reservoir to a syringe using a peristaltic pump. In another embodiment, the operation of the syringe filling station can begin by installing an administration cartridge into the filling station. The tubing **40** is typically threaded around the pump. The lower end of the tubing, also referred to as the proximal end is connected to a fitting for the canula. The upper part of the tubing **40** is positioned in place using hold down clamps **42** and is threaded through the air in-line sensor **55.** A reservoir is connected to the spike and the reservoir is then removably positioned using the reservoir attachment. A syringe is then inserted into the syringe retaining guide so that the canula is at least partially inserted into the syringe, and the syringe is releasably positioned within the filling station. The operator can then use the interface to specify a desired amount of fluid to dispense into the reservoir.

The following description illustrates some of the possible command functions that are available at the user interface. When a new administration cartridge is installed into the filling station, the control unit (processor) can automatically compensate for the volume of fluid contained within the cartridge. If a new reservoir is added, the control unit can compensate for the air in the line that can be present from the reservoir to the air in-line sensor. Alternatively, the interface can include a "PURGE" function to remove air from within the administration cartridge. In operation, a user can push and hold the PURGE button to operate the pump at a low speed to remove the air. After the system has been purged, the PURGE button may be released.

As stated above, the syringe retaining guide can also include a sensor to detect when a new syringe has been inserted. When a new syringe is inserted into the filling station the user interface can be programmed to display a reading indicating the insertion of a new syringe. An exemplary display could state, for example, "NEW SYRINGE LOADED." The operator could push this button to reset the Current Volume display. Alternatively, the control unit can be programmed to automatically reset the Current Volume to zero. For frequently repeated operations the control unit could store the syringe volume so that there would be no need to change the "FILL TO." However, an operator can still manually enter a desired fill volume. Typically, when the volume displayed in the "FILL TO" volume exceeds the "CURRENT VOLUME," a start button is enabled. The start button can display a command function such as "BEGIN FILL." The interface can also include command functions such as "CLEAR" to reset the fill volume.

In one embodiment, after the "BEGIN FILL" button has been activated by the operator, the pump will begin operating. The interface can include a "PAUSE" command function that can halt the operation. In yet another embodiment, touchscreen or input buttons can have multifunctions depending on the operation currently being carried out by the filling station. For example, if a touchscreen is being utilized, the "NEW SYRINGE LOADED" can be converted into a "PAUSE" button after pumping operations have started. During syringe filling, the CURRENT VOLUME" display can show the amount of fluid dispensed in real time. Additionally, if the "PAUSE" button has been pushed, the "BEGIN FILL" button can be reset to display a "RESUME FILL" button. After the specified volume has been dispensed, the control unit will stop the pump, and an "ADD MORE" button can be displayed to give an operator the opportunity to dispense additional fluid into the syringe. It should be recognized that the above examples are only exemplary of some of the command functions that can be incorporated into the control unit and the user interface.

From the foregoing discussion, it should be evident that the invention provides an apparatus and method that can be used to efficiently and accurately fill a syringe with a desired amount of fluid. The syringe filling station can include a user interface that allows an operator to easily control the amount of fluid dispensed into the syringe.

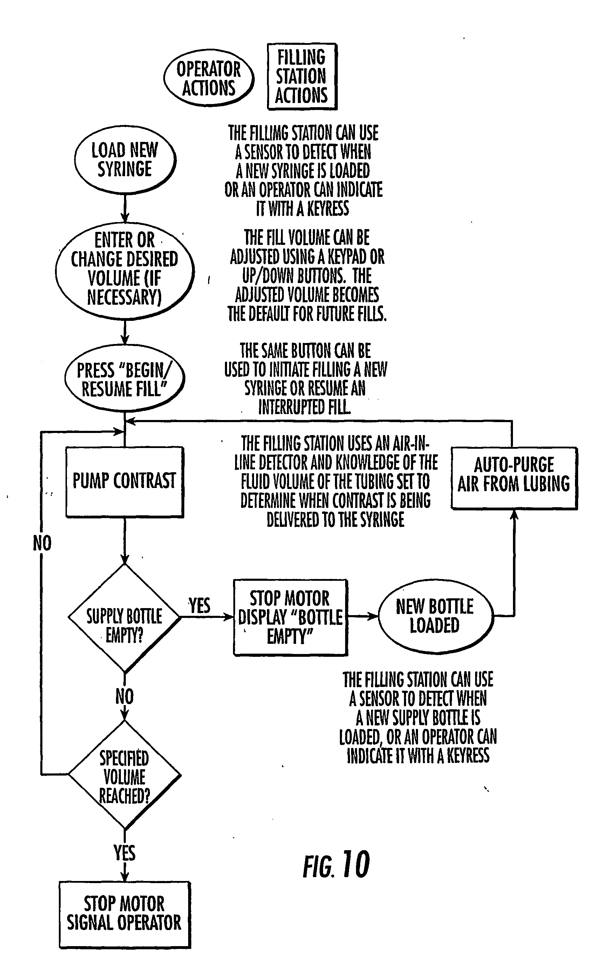

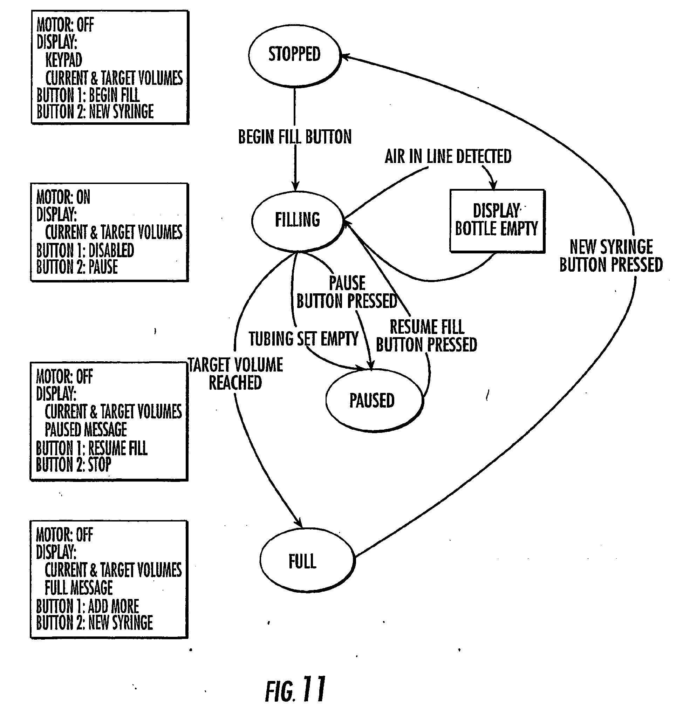

**FIGS. 10** and **11** illustrate flow diagrams describing possible steps the syringe filling station can take in filling a syringe. **FIG.10** depicts possible process steps that might occur in filling the syringe. In particular, **FIG. 10** illustrates possible functions that may be available at each step of the process. **FIG.11** correlates the operations being performed by the syringe filling station with pump status and possible command functions that may be available at the user interface. It should be recognized that different and additional command functions and operations are possible, and that the invention is not limited to any specific set of commands or operations.

In one exemplary embodiment, the syringe filling station is adeptly suited for receiving and filling syringes with a contrast medium that is useful in medical imaging and diagnostic testing. Such diagnostic imaging includes, but is not limited to, CT, NMR/MRI, Ultrasound, Fluoroscopy, PET, and the like. Injection syringes for performing diagnostic testing typically have volumes from about 10 to 500 cc, with a volume from about 50 to 400 cc being somewhat more typical. Preferably, the syringe volume is from about 100 to 200 cc. Syringe length is typically from about 2 to 30 cm, and syringe diameter is typically from about 0.2 to 5 cm.

Modifications and other embodiments of the inventions set forth herein will come to mind to one skilled in the art to which these inventions pertain having the benefit of the teachings presented in the foregoing descriptions and the associated drawings. Therefore, it is to be understood that the inventions are not to be limited to the specific embodiments disclosed and that modifications and other embodiments are intended to be included within the scope of the appended claims. Although specific terms are employed herein, they are used in a generic and descriptive sense only and not for purposes of limitation. Also, the invention may suitably comprise, consist of or consist essentially of the elements described herein, and the elements described herein may be practiced in the absence of any element which is or is not specifically disclosed herein.

Throughout the description, where embodiments are described as having, including, or comprising specific components, or where methods are described as having, including, or comprising specific steps, it is contemplated that embodiments of the present invention also consist essentially of, or consist of the recited components, and that the methods of the present invention also consist essentially of, or consist of the recited method steps. Further, it should be understood that the order of steps or order for performing certain actions may vary and need not be performed pursuant to a specific non-varying sequence so long as the invention remains operable. Moreover, two or more steps or actions may be conducted simultaneously.

## Claims

1. A device for delivering a fluid into a syringe (25) comprising:
a) a reservoir (15) attachment (17) for removably receiving a reservoir having a fluid;
b) a syringe retaining guide (50) for releasably securing a syringe;
c) a replaceable cartridge defining a fluid pathway between a reservoir and a syringe, said cartridge comprising a projecting member (35) for inserting into the reservoir, a flexible tubing (40) having a distal end attached to the projecting member, and a canula (45) attached to the proximal end of the flexible tubing, said canula adapted for at least partial insertion into a syringe; and
d) a pump (20) for pumping said fluid from a reservoir through said cartridge into a syringe.

2. The device according to claim 1, wherein the pump is a peristaltic pump.

3. The device according to claim 1, wherein the projecting member is in the form of a spike.

4. The device according to claim 1, wherein said projecting member is vented.

5. The device according to claim 1, further comprising a user interface for operating the device, the interface having input buttons and a display screen.

6. The device according to claim 4, further comprising: an air in- line sensor for detecting air in the flexible tubing.

7. The device according to claim 5, wherein the sensor is in communication with the user interface.

8. The device according to claim 4, wherein an operator can use the input buttons to specify the amount of volume to be dispensed into a syringe.

9. The device according to claim 1, wherein the cartridge is disposable.

10. The device according to claim 1, wherein the syringe retaining guide includes a guide sleeve for aligning the syringe with the canula.

11. The device according to claim 10, further comprising a canula mount and canula guide adapted to provide an aseptic interface between said canula and a syringe tip.

12. The device according to claim 1, further comprising a remote interface in communication with the pump, whereby said remote interface can control the pump so that the pump moves a specific volume of fluid.

13. The device according to claim 1, further comprising a syringe heater for warming a fluid disposed in the syringe.

14. The device according to claim 1, further comprising:
a reservoir having a fluid medium;
the projecting member inserted into the reservoir,
a syringe having a generally elongated body and a passageway disposed near the discharge region of the syringe, the canula extending into said passageway whereby fluid can be delivered from the reservoir into the syringe; and
a control unit that is operatively connected to the pump, whereby the control unit can control the pump so that a specific volume of fluid is dispensed into the syringe.

15. A device according to claim 14, wherein the reservoir is a bottle, pouch, bag, or container.

16. A device according to claim 14, wherein the fluid medium is a contrast medium, saline medium, or flushing medium.

17. A device according to claim 14, wherein the control unit further includes a processor and a user interface.

18. A device according to claim 17, wherein the user interface further comprises a touchscreen.

19. A device according to claim 17, wherein the user interface includes command function buttons for operating the device.

20. A device according to claim 17, wherein the control unit is in communication with a plurality of sensors for monitoring the device.

21. A device according to claim 20, wherein the sensors include at least one of the following: an air in-line sensor; a reservoir depletion sensor; a new syringe inserted sensor, or a new cartridge sensor.

22. A device according to claim 14, wherein the control unit can measure the amount of fluid dispensed into the syringe.

23. A device according to claim 22, wherein the control unit includes program code adapted to instruct the pump to dispense a specified volume of fluid into the syringe.

24. A device according to claim 23, wherein the program code will instruct the pump to cease pumping in the event that a sensor detects air in the cartridge.

25. A device according to claim 23, wherein the program code is adapted to instruct the pump to purge air from the cartridge in the event a sensor detects a new cartridge in the device.

26. A device according to claim 1, wherein the syringe retaining guide includes a guide sleeve and a gripping clamp, the guide sleeve adapted for aligning the syringe with the canula, and the gripping clamp adapted for releasably retaining the syringe.

27. A device according to claim 14, wherein a gap exists between the canula and the syringe.

28. A device according to claim 14, wherein the device provides a sterile fluid conduit from the reservoir to the syringe.

29. A device according to claim 14, wherein the cartridge is a disposable cartridge.

30. A device according to claim 14, further including a syringe support shelf disposed below the syringe retaining guide.

31. A device according to claim 30, wherein the syringe support shelf is moveable.

32. A device according to claim 14, further comprising a printer.

33. A device according to claim 32, wherein the printer is adapted to print labels containing Date, Time, Person Filling, Patient Name, Patient ID Number, Medical Name of Fluid Filled in Syringe, Brand Name of Fluid Filled in Syringe, Lot Number of Fluid Filled in Syringe, Volume Filled in Syringe, Warnings, or Cautions, or combinations thereof.

34. A device according to claim 14, wherein the device is in communication and operatively connected with an external interface adapted for controlling device functions.

35. A device according to claim 34, wherein the external interface is adapted to input the volume of fluid to dispense in a syringe.

36. A method of filling a syringe with a fluid comprising using a device according to any of claims 1-35:
a) connecting a reservoir to a fluid administration cartridge;
b) positioning a canula of the fluid administration cartridge at least partially in a syringe; and
c) moving a fluid from the reservoir to a syringe using a peristaltic pump.

37. A method of filling a syringe according to claim 36, further comprising the step of positioning the syringe on a movable support shelf.

38. A method of filling a syringe according to claim 36, further comprising the step of specifying an amount of fluid to move to the syringe.

39. A method of filling a syringe according to claim 38, wherein specifying an amount of fluid to dispense includes a user interface to input the amount of fluid.

40. A method of filling a syringe according to claim 36, further comprising the step of detecting the administration cartridge.

41. A method of filling a syringe according to claim 40, further including the step of purging air from the administration cartridge.

42. A method of filling a syringe according to claim 40, further including the step of compensating for air in the administration cartridge.

43. A method of filling a syringe according to claim 36, further including the step of measuring the amount of fluid that has been dispensed into the syringe.

44. A method of filling a syringe according to claim 43, wherein the step of measuring the amount of fluid includes a control unit.

45. A method of filling a syringe according to claim 44, wherein the pump and the control unit are in communication with each other.

46. A method of filling a syringe according to claim 36, further comprising the step of heating the fluid disposed in the reservoir.

47. A method of filling a syringe with a fluid according to claim 36, further comprising:
a) providing a filling station having a reservoir attachment for removably receiving the reservoir, the pump, and a syringe retaining guide;
b) providing the fluid cartridge with a projecting member, the canula, and a flexible tubing;
c) installing the cartridge into the filling station to define a fluid pathway from the reservoir attachment to the syringe retaining guide;
d) connecting the reservoir to the reservoir attachment; and
e) inserting the syringe into the retaining guide so that at least a portion of the canula is partially inserted into the syringe

48. A method of filling a syringe according to claim 47, wherein the step of pumping the fluid further includes the step of inputing a specified volume of fluid to pump into a user interface.

49. A method of filling a syringe according to claim 48, further comprising communicating the specified volume to a control unit.

50. A method of filling a syringe according to claim 49, further including sending commands from the control unit to the pump, whereby the pump moves a specified volume of fluid from the reservoir to the syringe.

51. A method of filling a syringe according to claim 47, further comprising the step of detecting air in the cartridge.

52. A method of filling a syringe according to claim 51, further comprising the step of purging air from the cartridge.

53. A method of filling a syringe according to claim 49, further including the step of displaying the volume of fluid moved to the syringe.

54. A method of filling a syringe according to claim 49, further comprising calculating the volume of fluid moved from the reservoir to said pump.

55. A method of filling a syringe according to claim 52, further comprising a processor for calculating the volume of fluid moved from the reservoir to the pump.

## Patentansprüche

1. Vorrichtung zum Zuführen eines Fluids in eine Spritze (25) mit:
a) einer Befestigung (17) für ein Reservoir (15) zum herausnehmbaren Aufnehmen eines Reservoirs, das ein Fluid aufweist;
b) einer Spritzenhalteführung (50) zum lösbaren Sichern einer Spritze;
c) einer auswechselbaren Kartusche, die einen Fluidpfad zwischen einem Reservoir und einer Spritze definiert, wobei die Kartusche ein vorspringendes Element (35) zum Einführen in das Reservoir, einen flexiblen Schlauch (40), der ein distales Ende aufweist, das an dem vorspringenden Element angebracht ist, und eine Kanüle (45) aufweist, die an dem proximalen Ende des flexiblen Schlauchs angebracht ist, wobei die Kanüle für ein zumindest teilweises Einführen in eine Spritze ausgelegt ist; und
d) einer Pumpe (20) zum Pumpen des Fluids von einem Reservoir durch die Kartusche in eine Spritze.

2. Vorrichtung nach Anspruch 1, wobei die Pumpe eine Peristaltikpumpe ist.

3. Vorrichtung nach Anspruch 1, wobei das vorspringende Element in der Form eines Dorns vorliegt.

4. Vorrichtung nach Anspruch 1, wobei das vorspringende Element belüftet ist.

5. Vorrichtung nach Anspruch 1, die des Weiteren eine Benutzerschnittstelle zum Betätigen der Vorrichtung aufweist, wobei die Schnittstelle Eingabetasten und einen Anzeigebildschirm aufweist.

6. Vorrichtung nach Anspruch 4, die des Weiteren einen Luftblasensensor (Luft-in-der-Leitung-Sensor) zum Erfassen von Luft in dem flexiblen Schlauch aufweist.

7. Vorrichtung nach Anspruch 5, wobei sich der Sensor in Kommunikation mit der Benutzerschnittstelle befindet.

8. Vorrichtung nach Anspruch 4, wobei eine Bedienperson die Eingabetasten benutzen kann, um die Größe des Volumens zu spezifizieren, das in eine Spritze abgegeben werden soll.

9. Vorrichtung nach Anspruch 1, wobei die Kartusche wegwerfbar ist.

10. Vorrichtung nach Anspruch 1, wobei die Spritzenhalteführung eine Führungsbuchse zum Ausrichten der Spritze in Bezug auf die Kanüle umfasst.

11. Vorrichtung nach Anspruch 10, die des Weiteren eine Kanülenhalterung und eine Kanülenführung aufweist, die dafür ausgelegt sind, eine aseptische Schnittstelle zwischen der Kanüle und einer Spritzenspitze vorzusehen.

12. Vorrichtung nach Anspruch 1, die des Weiteren eine Fernschnittstelle in Kommunikation mit der Pumpe aufweist, wodurch die Fernschnittstelle die Pumpe so steuern kann, dass die Pumpe ein spezifisches Volumen an Fluid bewegt.

13. Vorrichtung nach Anspruch 1, die des Weiteren ein Spritzenheizgerät zum Erwärmen eines Fluids, das sich in der Spritze befindet, aufweist.

14. Vorrichtung nach Anspruch 1, des Weiteren mit:
einem Reservoir, das ein Fluidmedium aufweist;
wobei das vorspringende Element in das Reservoir eingeführt ist;
einer Spritze, die einen allgemein länglichen Körper und einen Durchgang aufweist, der sich nahe dem Auslassbereich der Spritze befindet, wobei sich die Kanüle in den Durchgang erstreckt, wodurch Fluid von dem Reservoir in die Spritze zugeführt werden kann; und
einer Steuereinheit, die operativ mit der Pumpe verbunden ist, wodurch die Steuereinheit die Pumpe so steuern kann, dass ein spezifisches Volumen an Fluid in die Spritze abgegeben wird.

15. Vorrichtung nach Anspruch 14, wobei das Reservoir eine Flasche, ein Beutel, eine Tasche oder ein Behälter ist.

16. Vorrichtung nach Anspruch 14, wobei das Fluidmedium ein Kontrastmedium, ein Salzlösungsmedium oder ein Spülmedium ist.

17. Vorrichtung nach Anspruch 14, wobei die Steuereinheit des Weiteren einen Prozessor und eine Benutzerschnittstelle umfasst.

18. Vorrichtung nach Anspruch 17, wobei die Benutzerschnittstelle des Weiteren ein Touchscreen aufweist.

19. Vorrichtung nach Anspruch 17, wobei die Benutzerschnittstelle Befehlsfunktionstasten zum Betätigen der Vorrichtung umfasst.

20. Vorrichtung nach Anspruch 17, wobei die Steuereinheit in Kommunikation mit einer Mehrzahl von Sensoren zum Überwachen der Vorrichtung steht.

21. Vorrichtung nach Anspruch 20, wobei die Sensoren wenigstens einen von den Folgenden umfassen: einen Luftblasensensor (Luft-in-der-Leitung-Sensor); einen Reservoir-Depletions-Sensor; einen Neue-Spritze-ist-eingeführt-Sensor oder einen Neue-Kartusche-Sensor.

22. Vorrichtung nach Anspruch 14, wobei die Steuereinheit die Fluidmengemessen kann, die in die Spritze abgegeben wird.

23. Vorrichtung nach Anspruch 22, wobei die Steuereinheit einen Programmcode umfasst, der dafür ausgelegt ist, die Pumpe zu instruieren, ein spezifiziertes Volumen an Fluid in die Spritze abzugeben.

24. Vorrichtung nach Anspruch 23, wobei der Programmcode die Pumpe instruieren wird, mit dem Pumpen in dem Fall aufzuhören, dass ein Sensor Luft in der Kartusche entdeckt.

25. Vorrichtung nach Anspruch 23, wobei der Programmcode dafür ausgelegt ist, die Pumpe zu instruieren, Luft aus der Kartusche in dem Fall abzusaugen, dass ein Sensor eine neue Kartusche in der Vorrichtung entdeckt.

26. Vorrichtung nach Anspruch 1, wobei die Spritzenhalteführung eine Führungsbuchse und eine Einspannklemme umfasst, wobei die Führungsbuchse dafür ausgelegt ist, die Spritze in Bezug auf die Kanüle auszurichten, und die Einspannklemme dafür ausgelegt ist, die Spritze lösbar zu halten.

27. Vorrichtung nach Anspruch 14, wobei ein Spalt zwischen der Kanüle und der Spritze existiert.

28. Vorrichtung nach Anspruch 14, wobei die Vorrichtung einen sterilen Fluidkanal von dem Reservoir zu der Spritze vorsieht.

29. Vorrichtung nach Anspruch 14, wobei die Kartusche eine Wegwerf-Kartusche ist.

30. Vorrichtung nach Anspruch 14, die des Weiteren ein Spritzenstützgestell enthält, das unterhalb der Spritzenhalteführung angeordnet ist.

31. Vorrichtung nach Anspruch 30, wobei das Spritzenstützgestell beweglich ist.

32. Vorrichtung nach Anspruch 14, die des Weiteren einen Drucker aufweist.

33. Vorrichtung nach Anspruch 32, wobei der Drucker dafür ausgelegt ist, Etiketten zu drucken, die Folgendes enthalten: Datum, Zeit, Person, die das Füllen vornimmt, Patientenname, Patienten-ID-Nummer, medizinischer Name des Fluids, das in die Spritze gefüllt wird, Handelsmarkenname des Fluids, das in die Spritze gefüllt wird, Chargennummer des Fluids, das in die Spritze gefüllt wird, Volumen, das in die Spritze gefüllt wird, Warnhinweise oder Vorsichtsmaßregeln, oder Kombinationen davon.

34. Vorrichtung nach Anspruch 14, wobei die Vorrichtung in Kommunikation mit einer externen Schnittstelle steht, die dafür ausgelegt ist, Vorrichtungsfunktionen zu steuern, und mit dieser externen Schnittstelle operativ verbunden ist.

35. Vorrichtung nach Anspruch 34, wobei die externe Schnittstelle dafür ausgelegt ist, das Volumen an Fluid einzugeben, das in eine Spritze abgegeben werden soll.

36. Verfahren zum Befüllen einer Spritze mit einem Fluid unter Verwendung einer Vorrichtung nach einem der Ansprüche 1 bis 35, wobei das Verfahren Folgendes umfasst:
a) Verbinden eines Reservoirs mit einer Fluidzufuhrkartusche;
b) Positionieren einer Kanüle der Fluidzufuhrkartusche zumindest teilweise in einer Spritze; und
c) Bewegen eines Fluids von dem Reservoir zu einer Spritze unter Verwendung einer Peristaltikpumpe.

37. Verfahren zum Befüllen einer Spritze nach Anspruch 36, das des Weiteren den Schritt des Positionierens der Spritze auf einem beweglichen Stützgestell umfasst.

38. Verfahren zum Befüllen einer Spritze nach Anspruch 36, das des Weiteren den Schritt des Spezifizierens einer Fluidmenge umfasst, die zu der Spritze bewegt werden soll.

39. Verfahren zum Befüllen einer Spritze nach Anspruch 38, wobei das Spezifizieren einer Fluidmenge, die abgegeben werden soll, eine Benutzerschnittstelle zum Eingeben des Betrags an Fluid umfasst.

40. Verfahren zum Befüllen einer Spritze nach Anspruch 36, das des Weiteren den Schritt des Erfassens der Zufuhrkartusche umfasst.

41. Verfahren zum Befüllen einer Spritze nach Anspruch 40, das des Weiteren den Schritt des Absaugens von Luft aus der Zufuhrkartusche umfasst.

42. Verfahren zum Befüllen einer Spritze nach Anspruch 40, das des Weiteren den Schritt des Kompensierens von Luft in der Zufuhrkartusche umfasst.

43. Verfahren zum Befüllen einer Spritze nach Anspruch 36, das des Weiteren den Schritt des Messens der Fluidmenge umfasst, die in die Spritze abgegeben worden ist.

44. Verfahren zum Befüllen einer Spritze nach Anspruch 43, wobei der Schritt des Messens der Fluidmenge eine Steuergeinheit umfasst.

45. Verfahren zum Befüllen einer Spritze nach Anspruch 44, wobei die Pumpe und die Steuereinheit in Kommunikation miteinander stehen.

46. Verfahren zum Befüllen einer Spritze nach Anspruch 36, das des Weiteren den Schritt des Erwärmens des Fluids, das sich in dem Reservoir befindet, umfasst.

47. Verfahren zum Befüllen einer Spritze mit Fluid nach Anspruch 36, das des Weiteren Folgendes umfasst:
a) Vorsehen einer Füllstation, die eine Reservoirhalterung zum herausnehmbaren Aufnehmen des Reservoirs, die Pumpe und eine Spritzenhalteführung aufweist;
b) Vorsehen der Fluidkartusche mit einem vorspringenden Element, der Kanüle und einem flexiblen Schlauch;
c) Installieren der Kartusche in der Füllstation, um einen Fluidpfad von der Reservoirhalterung zu der Spritzenhalteführung zu definieren;
d) Verbinden des Reservoirs mit der Reservoirhalterung; und
e) Einführen der Spritze in die Halteführung derart, dass wenigstens ein Teil der Kanüle zum Teil in die Spritze eingeführt ist.

48. Verfahren zum Befüllen einer Spritze nach Anspruch 47, wobei der Schritt des Pumpens des Fluids des Weiteren den Schritt des Eingebens eines spezifizierten Volumens an Fluid, das gepumpt werden soll, in eine Benutzerschnittstelle umfasst.

49. Verfahren zum Befüllen einer Spritze nach Anspruch 48, das des Weiteren das Kommunizieren des spezifizierten Volumens zu einer Steuereinheit umfasst.

50. Verfahren zum Befüllen einer Spritze nach Anspruch 49, das des Weiteren das Senden von Befehlen von der Steuereinheit zu der Pumpe umfasst, wodurch die Pumpe ein spezifiziertes Volumen an Fluid von dem Reservoir zu der Spritze bewegt.

51. Verfahren zum Befüllen einer Spritze nach Anspruch 47, das des Weiteren den Schritt des Erfassens von Luft in der Kartusche umfasst.

52. Verfahren zum Befüllen einer Spritze nach Anspruch 51, das des Weiteren den Schritt des Absaugens von Luft aus der Kartusche umfasst.

53. Verfahren zum Befüllen einer Spritze nach Anspruch 49, das des Weiteren den Schritt des Anzeigens des Volumens an Fluid, das in die Spritze bewegt wird, umfasst.

54. Verfahren zum Befüllen einer Spritze nach Anspruch 49, das des Weiteren das Berechnen des Volumens an Fluid, das von dem Reservoir zu der Pumpe bewegt wird, umfasst.

55. Verfahren zum Befüllen einer Spritze nach Anspruch 52, das des Weiteren einen Prozessor zum Berechnen des Volumens an Fluid, das von dem Reservoir zu der Pumpe bewegt wird, umfasst.

## Revendications

1. Dispositif destiné à distribuer un fluide dans une seringue (25) comprenant :
a) une fixation (17) de réservoir (15) destinée à recevoir amovible un réservoir présentant un fluide ;
b) un guide de retenue de seringue (50) destiné à fixer libérable une seringue ;
c) une cartouche remplaçable définissant un trajet de fluide entre un réservoir et une seringue, ladite cartouche comprenant un élément en saillie (35) destiné à être inséré dans le réservoir, un tube flexible (40) présentant une extrémité distale fixée à l'élément en saillie, et une canule (45) fixée à l'extrémité proximale du tube flexible, ladite canule étant conçue pour être au moins en partie insérée dans une seringue ; et
d) une pompe (20) destinée à pomper ledit fluide d'un réservoir dans une seringue en passant par ladite cartouche.

2. Dispositif selon la revendication 1, dans lequel la pompe est une pompe péristaltique.

3. Dispositif selon la revendication 1, dans lequel l'élément en saillie présente la forme d'une pointe.

4. Dispositif selon la revendication 1, dans lequel ledit élément en saillie est mis à l'air libre.

5. Dispositif selon la revendication 1, comprenant en outre une interface utilisateur destinée à faire fonctionner le dispositif, l'interface présentant des boutons de saisie et un écran.

6. Dispositif selon la revendication 4, comprenant en outre un capteur d'air en ligne destiné à détecter l'air dans le tube flexible.

7. Dispositif selon la revendication 5, dans lequel le capteur est en communication avec l'interface utilisateur.

8. Dispositif selon la revendication 4, dans lequel un opérateur peut utiliser les boutons de saisie pour spécifier la quantité de volume destinée à être distribuée dans une seringue.

9. Dispositif selon la revendication 1, dans lequel la cartouche est jetable.

10. Dispositif selon la revendication 1, dans lequel le guide de retenue de seringue comprend un manchon de guidage destiné aligner la seringue avec la canule.

11. Dispositif selon la revendication 10, comprenant en outre un support de canule et un guide de canule conçus pour fournir une interface aseptique entre ladite canule et une pointe de seringue.

12. Dispositif selon la revendication 1, comprenant en outre une interface distante en communication avec la pompe, ladite interface distante pouvant commander la pompe de sorte que la pompe déplace un volume spécifique de fluide.

13. Dispositif selon la revendication 1, comprenant en outre un dispositif de chauffage de seringue destiné à réchauffer un fluide disposé dans la seringue.

14. Dispositif selon la revendication 1, comprenant en outre :
un réservoir présentant un milieu fluide ;
l'élément en saillie étant inséré dans le réservoir,
une seringue présentant un corps généralement allongé et un passage disposé à proximité de la zone d'évacuation de la seringue, la canule s'étendant dans ledit passage, le fluide pouvant être distribué du réservoir dans la seringue ; et
une unité de commande reliée fonctionnellement à la pompe, l'unité de commande pouvant commander la pompe de sorte qu'un volume spécifique du fluide est distribué dans la seringue.

15. Dispositif selon la revendication 14, dans lequel le réservoir est une bouteille, une poche, un sac, ou un contenant.

16. Dispositif selon la revendication 14, dans lequel le milieu fluide est un produit de contraste, un milieu salin, ou un milieu de rinçage.

17. Dispositif selon la revendication 14, dans lequel l'interface utilisateur comprend en outre un processeur et une interface utilisateur.

18. Dispositif selon la revendication 17, dans lequel l'interface utilisateur comprend en outre un écran tactile.

19. Dispositif selon la revendication 17, dans lequel l'interface utilisateur comprend des boutons à fonction de commande destinés à faire fonctionner le dispositif.

20. Dispositif selon la revendication 17, dans lequel l'unité de commande est en communication avec une pluralité de capteurs destinés à surveiller le dispositif.

21. Dispositif selon la revendication 20, dans lequel les capteurs comprennent au moins un capteur parmi les capteurs suivants : un capteur d'air en ligne ; un capteur d'épuisement de réservoir ; un capteur de nouvelle seringue insérée, ou un capteur de nouvelle cartouche.

22. Dispositif selon la revendication 14, dans lequel l'unité de commande peut mesurer la quantité de fluide distribuée dans la seringue.

23. Dispositif selon la revendication 22, dans lequel l'unité de commande comprend un code de programme conçu pour donner l'instruction à la pompe de distribuer un volume spécifié de fluide dans la seringue.

24. Dispositif selon la revendication 23, dans lequel le code de programme donnera l'instruction à la pompe de cesser le pompage au cas où un capteur détecte l'air dans la cartouche.

25. Dispositif selon la revendication 23, dans lequel le code de programme est conçu pour donner l'instruction à la pompe de purger l'air de la cartouche au cas où un capteur détecte une nouvelle cartouche dans le dispositif.

26. Dispositif selon la revendication 1, dans lequel le guide de retenue de seringue comprend un manchon de guidage et une pince de préhension, le manchon de guidage étant conçu pour aligner la seringue avec la canule, et la pince de préhension étant conçue pour retenir amovible la seringue.

27. Dispositif selon la revendication 14, dans lequel il existe un espace entre la canule et la seringue.

28. Dispositif selon la revendication 14, dans lequel le dispositif fournit une conduite de fluide stérile allant du réservoir à la seringue.

29. Dispositif selon la revendication 14, dans lequel la cartouche est une cartouche jetable.

30. Dispositif selon la revendication 14, comprenant en outre une tablette de support de seringue disposée sous le guide de retenue de seringue.

31. Dispositif selon la revendication 30, dans lequel la tablette de support de seringue est mobile.

32. Dispositif selon la revendication 14, comprenant en outre une imprimante.

33. Dispositif selon la revendication 32, dans lequel l'imprimante est conçue pour imprimer des étiquettes sur lesquelles figurent la date, l'heure, le nom de la personne effectuant le remplissage, le nom du patient, le numéro d'identification du patient, le nom médical du fluide remplissant la seringue, le nom commercial du fluide remplissant la seringue, le numéro de lot du fluide remplissant la seringue, le volume versé dans la seringue, les avertissements, ou précautions, ou leurs combinaisons.

34. Dispositif selon la revendication 14, dans lequel le dispositif est en communication avec une interface externe conçue pour commander les fonctions du dispositif et relié fonctionnellement à celle-ci.

35. Dispositif selon la revendication 34, dans lequel l'interface externe est conçue pour saisir le volume de fluide à distribuer dans une seringue.

36. Procédé de remplissage d'une seringue avec un fluide au moyen d'un dispositif selon l'une quelconque des revendications 1 à 35 comprenant :
a) le raccordement d'un réservoir à une cartouche d'administration de fluide ;
b) le positionnement d'une canule de la cartouche d'administration de fluide au moins en partie dans une seringue ; et
c) le déplacement d'un fluide du réservoir vers une seringue au moyen d'une pompe péristaltique.

37. Procédé de remplissage d'une seringue selon la revendication 36, comprenant en outre l'étape de positionnement de la seringue sur une tablette de support mobile.

38. Procédé de remplissage d'une seringue selon la revendication 36, comprenant en outre l'étape de spécification d'une quantité de fluide à déplacer.

39. Procédé de remplissage d'une seringue selon la revendication 38, dans lequel la spécification d'une quantité de fluide à distribuer comprend une interface utilisateur pour saisir la quantité de fluide.

40. Procédé de remplissage d'une seringue selon la revendication 36, comprenant en outre l'étape de détection de la cartouche d'administration.

41. Procédé de remplissage d'une seringue selon la revendication 40, comprenant en outre l'étape de purge d'air de la cartouche d'administration.

42. Procédé de remplissage selon la revendication 40, comprenant en outre l'étape de compensation de l'air dans la cartouche d'administration.

43. Procédé de remplissage d'une seringue selon la revendication 36, comprenant en outre l'étape de mesure de la quantité de fluide qui a été distribuée dans la seringue.

44. Procédé de remplissage d'une seringue selon la revendication 43, dans lequel l'étape de mesure de la quantité de fluide comprend une unité de commande.

45. Procédé de remplissage d'une seringue selon la revendication 44, dans lequel la pompe et l'unité de commande sont en communication l'une avec l'autre.

46. Procédé de remplissage d'une seringue selon la revendication 36, comprenant en outre l'étape de chauffage du fluide disposé dans le réservoir.

47. Procédé de remplissage d'une seringue avec un fluide selon la revendication 36, comprenant en outre les étapes consistant à :
a) fournir un poste de remplissage présentant une fixation de réservoir destinée à recevoir amovible le réservoir, la pompe, et un guide de retenue de seringue ;
b) pourvoir la cartouche de fluide d'un élément en saillie, de la canule, et d'un tube flexible ;
c) installer la cartouche dans le poste de remplissage pour définir un trajet de fluide de la fixation de réservoir au guide de retenue de seringue ;
d) raccorder le réservoir à la fixation de réservoir ; et
e) insérer la seringue dans le guide de retenue de sorte qu'au moins une partie de la canule soit en partie insérée dans la seringue.

48. Procédé de remplissage d'une seringue selon la revendication 47, dans lequel l'étape de pompage du fluide comprend en outre l'étape de saisie d'un volume spécifié de fluide à pomper dans une interface utilisateur.

49. Procédé de remplissage selon la revendication 48, comprenant en outre l'étape consistant à faire communiquer le volume spécifié avec une unité de commande.

50. Procédé de remplissage d'une seringue selon la revendication 49, comprenant en outre l'envoi de commandes de l'unité de commande à la pompe, la pompe déplaçant un volume spécifié de fluide du réservoir à la seringue.

51. Procédé de remplissage d'une seringue selon la revendication 47, comprenant en outre l'étape de détection d'air dans la cartouche.

52. Procédé de remplissage d'une seringue selon la revendication 51, comprenant en outre l'étape de purge d'air de la cartouche.

53. Procédé de remplissage d'une seringue selon la revendication 49, comprenant en outre l'étape d'affichage du volume de fluide déplacé vers la seringue.

54. Procédé de remplissage d'une seringue selon la revendication 49, comprenant en outre le calcul du volume de fluide déplacé du réservoir à ladite pompe.

55. Procédé de remplissage d'une seringue selon la revendication 52, comprenant en outre un processeur de calcul du volume de fluide déplacé du réservoir à la pompe.
